# EUROPEAN PATENT APPLICATION

(11) **EP 1 642 573 A1**
(43) Date of publication of application: **05.04.2006**
(21) Application number: 05022671.1
(22) Date of filing: 23.04.2003
(51) Int. Cl.: A61K 31/05, A61P 1/04

(54) **Use of GABA B receptor modulators for treating gastro-intestinal disorders**

(30) Priority: 24.04.2002 GB 0209481
(62) Divisional of application: 03722530.7
(71) Applicant: Novartis AG, 4056 Basel (CH); Novartis Pharma GmbH, 1235 Vienna (AT)
(72) Inventor: Pfannkuche, Hans-Jürgen, 79576 Weil (DE)
(74) Representative: de Weerd, Petrus G.W.

(57) **Abstract**

The invention relates to the use of a compound acting as positive allosteric modulator of GABA_{B} receptors in the treatment of GERD, regurgitation, IBS, dyspepsia, postoperative complaints and conditions associated with visceral discomfort/pain.

## Description

The present invention relates to new pharmaceutical uses of compounds acting as positive allosteric modulators at γ-aminobutyric acid, type B (GABA_{B}) receptors. They are generically referred to hereinafter as GABA_{B} receptor modulators.

More particularly the invention relates to the use of GABA_{B} receptor modulators in certain gastro-intestinal disorders including gastro-esophageal reflux disease and conditions associated with visceral discomfort and pain.

GABA_{B} receptor modulators such as 2,6-Di-tert-butyl-4-(3-hydroxy-2,2-dimethyl-propyl)-phenol and its aldehyde analog are known for example from Urwyler S et al., (2001) Molecular Pharmacology, 60; 963-971.

Gastro-Esophageal Reflux Disease (GERD) is the most common ailment in the upper gastro-intestinal tract; its cardinal feature and symptom is commonly known as "heartburn". A major factor responsible of GERD is an incompetence of the Lower Esophageal Sphincter that opens transiently and allows passage of acidic material from the stomach into the esophagus. This motor event denominated Transient Lower Esophageal Sphincter Relaxation (TLESR) occurs more often in patients suffering from GERD than in healthy subjects and in infants with regurgitation. Current standard therapies in GERD aim at suppressing gastric acid secretion or enhancing gastrointestinal motility to limit the exposure of the esophagus to acidic gastric contents. Frequent exposure of the esophageal mucosa to acid can trigger pain (often perceived as heartburn) and lead to erosions in the esophagus. Todate, there is no treatment available which actually reduces the occurrence of TLESRs and, thereby, the symptoms of pain associated with GERD or regurgitation in infants.

Visceral pain and discomfort is not only a symptom of GERD. Patients suffering from Irritable Bowel Syndrome (IBS), dyspepsia, diseases of the biliary tract, pancreas, urinary bladder and postoperative conditions report pain and discomfort. Visceral hypersensitivity has been discovered as a key phenomenon in many patients suffering from diseases like IBS, dyspepsia, GERD and other conditions listed above. Todate, there is no treatment available which specifically treats visceral hypersensitivity and, thereby, reduces symptoms of pain and discomfort in patients suffering from GERD, IBS, dyspepsia, diseases of the biliary tract, pancreas, urinary bladder and postoperative conditions.

It has recently been reported e.g. in WO 98/11885 that substances acting as agonists at GABA_{B} receptors can reduce TLESRs.

GABA_{B} agonists such as Baclofen play a key role in nervous circuitries mediating TLESR. For example, GABA_{B} receptors are present in the nodose ganglion [S. Smid et al., Am. J. Physiol. (2001) 281: G1494-G1501], and the Dorsal Vagal Complex (DVC) [P. Brooks et al., J. Physiol. (1992) 457: 115-129; C. Mc Dermott et al., Gastroenterol. (2001) 120: 1749-1762]. Additionally, Baclofen has been shown to reduce the sensitivity of vagal afferent and efferent fibers [S. Smid et al., Am. J. Physiol. (2001) 281; G1494-G1501; D, Blackshaw, Br. J. Pharmacol. (2000) 130: 279-288].

Administration of Baclofen reduces the frequency of TLESR in ferrets, [L.A. Blackshaw et al., Am. J. Physiol. (1999) 277: G867-874, dogs [A. Lehmann et al., Gastroenterol, (1999) 117; 1147-1,L54J, in GERD patients [O. Zhang et al., Gut (2002) 50: 19-24] and healthy subjects [I. Lidums et al., Gastroenterol., (2000) 118: 7-13]. Furthermore, long-term recording of esophageal pH in GERD patients showed a significant reduction in esophageal acid exposure attributable to the reduction of TLESR by Baclofen [L. Cange, Alim. Pharmacol. & Ther. (2002) 16: 869-873].

In accordance with the present invention it has now surprisingly been found that substances acting as positive allosteric modulators at GABA_{B} receptors, but lacking classical GABA_{B} receptor agonist activity, show activity in animal models indicative for use in the treatment of the pathology of GERD, regurgitation, IBS, dyspepsia, and conditions associated with visceral pain and discomfort, as indicated in experiments including the following :

In the gastric distension-induced TLESR and crural diaphragm inhibition model in lightly sedated cats according to a modification of Liu J. et al., Am. J. Physiology (2002) 283:G1276-G1281, the receptors modulators at doses of 0.1-10 mg/kg (oral or parenteral administration) reduce the frequency of TLESR and block the inhibition of the crural diaphragm occurring concomitantly to the TLESR.

In the gastric distension-induced TLESR model in conscious dogs equipped with a chronic esophagostomy according to a modification of Lehmann A. et al., Gastroenterology (1999) 117:1147-1154, the receptors modulators at doses of 0.1-10 mg/kg (oral or parenteral administration) reduce the frequency of TLESR and reduce the esophageal acid exposure.

In the noxious colorectal distension model in the rat according to a modification of Morteau, O. et al., Dig. Dis. Sci. (1994) 39; 1239-1248 [for basics concerning the method, see Ness, T.J. and Gebhart, G.F., Brain Res. (1988) 450: 153-169; Gebharr, G.F. and Sengupta, J.N., in Gaginella, T.A., ed., Methods in gastrointestinal pharmacology, Boca Raton: CRC press (1996), 359-373], the receptor modulators at doses of 0.1-10 mg/kg (oral or parenteral administration) reduce responses to colorectal distension, such as pseudoaffective reflexes or abdominal striated muscle contractions, indicative of a visceral antinociceptive activity.

In the colorectal distension model of hyperalgesia in murine according to a modification of Traub, R.T. et al., Neurosci. (1996) 74: 873-884, the receptor modulators at doses of 0.1-10 mg/kg (oral or parenteral administration) inhibit the expression of immediate early genes following noxious stimulation of afferent fibers, again, indicative of a visceral antinociceptive intervention.

These findings with receptor modulators which lack classical GABA_{B} receptor agonist activity are indicative of therapeutic potential in the treatment of GERD, regurgitation, IBS, dyspepsia and of conditions associated with visceral discomfort/pain.

Moreover, it has been found that positive allosteric modulators at doses of 0.1-10 mglkg (parenteral adminstration) show activity in the post-operative ileus model according to Huge, A. et al., J. Surg. Res (1998) 74: 112-118, as evidenced by a faster restauration of gastrointestinal motility as compared to vehicle/placebo treatment.

Hence, it follows that positive allosteric modulators of the GABA_{B} receptor are useful in the treatment of post-operative complaints such as visceral pain/discomfort and ileus.

For the above-mentioned indications the appropriate dosage will of course vary depending upon, for example, the compound employed, the host, the mode of administration and the nature and severity of the condition being treated. However, in general, satisfactory results in animals are indicated to be obtained at a daily dosage of from about 0.1 to about 10 mg/kg body weight. In larger mammals, for example humans, an indicated daily dosage is in the range from about 1 to about 200 mg of a receptor modulator conveniently administered, for example, in divided doses up to four times a day.

The present invention accordingly provides the use of a receptor modulator in the treatment of the above-mentioned conditions.

For use according to the invention, the GABA_{B} receptor modulator may be administered as single active agent or in combination with other active agents, in any usual manner, e.g. orally, for example in the form of tablets or capsules, or parenterally, for example in the form of injection solutions or suspensions.

Moreover, the present invention provides a pharmaceutical composition comprising a GABA_{B} receptor modulator in association with at least one pharmaceutical carrier or diluent for use in the treatment of any of the above-indicated diseases. Such compositions may be manufactured in conventional manner. Unit dosage forms may contain, for example, from about 0.25 to about 50 mg of the receptor modulator.

The present invention also provides the use of a GABA_{B} receptor modulator for the manufacture of a pharmaceutical composition for the treatment of any of the above-indicated diseases.

The invention furthermore provides a method for the treatment of any of the above-indicated diseases, in a subject in need of such treatment, which comprises administering to said subject a therapeutically effective amount of a GABA_{B} receptor modulator.

## Claims

1. The use of a compound acting as positive allosteric modulator of GABA_{B} receptors (a GABA_{B} receptor modulator) in the treatment of GERD, regurgitation, IBS, dyspepsia, postoperative complaints and conditions associated with visceral discomfort/pain.

2. The use of a GABA_{B} receptor modulator for the manufacture of a pharmaceutical composition for the treatment of GERD, regurgitation, IBS, dyspepsia, postoperative complaints and conditions associated with visceral discomfort/pain.

3. A pharmaceutical composition comprising a GABA_{B} receptor modulator in association with at least one pharmaceutical carrier or diluent, for use in the treatment of GERD, regurgitation, IBS, dyspepsia, postoperative complaints and conditions associated with visceral discomfort/pain.

4. A method for treating GERD, regurgitation, IBS, dyspepsia, postoperative complaints and conditions associated with visceral discomfort/pain in a subject in need of such treatment, which comprises administering to said subject a therapeutically effective amount of a GABA_{B} receptor modulator.
